# EUROPÄISCHE PATENTANMELDUNG

(11) **EP 4 751 689 A1**
(43) Veröffentlichungstag der Anmeldung: **03.06.2026**
(21) Anmeldenummer: 24216700.5
(22) Anmeldetag: 30.11.2024
(51) Int. Cl.: A61F 5/56

(54) **ZAHNSCHIENE**

(71) Anmelder: Alwaflex AG, 5430 Wettingen (CH)
(72) Erfinder: Alraun, Egon, 5426 Lengnau (CH)
(74) Vertreter: Fenner, Seraina

(57) **Zusammenfassung**

Zahnschiene (1) zum Arretieren des Unterkiefers, vorzugsweise in einem vordefinierten Protrusions-Verhältnis von Ober- und Unterkiefer, beinhaltend eine Oberkieferschale (2), eine Unterkieferschale (3) und einen Verbindungssteg (4), wobei die Ober- und Unterkieferschale über den Verbindungssteg fest miteinander verbunden sind, wobei die Zahnschiene derart ausgebildet ist, dass im Bereich der Kauflächen (6) Speichelkanäle (5) zwischen der Zahnschiene und der Zahnkaufläche vorliegen, die eine permanente Speichelunterspülung der Zahnschiene während des Tragens der Zahnschiene beim Schlafen gewährleisten.

## Beschreibung

Die Erfindung betrifft eine Zahnschiene zum Arretieren des Unterkiefers, vorzugsweise in einem vordefinierten Protrusions-Verhältnis von Ober- und Unterkiefer beinhaltend, eine Oberkieferschale, eine Unterkieferschale und einen Verbindungssteg, wobei die Ober- und Unterkieferschale über den Verbindungssteg fix miteinander verbunden sind.

Zahnschienen werden meist zur Behandlung von Schnarchen, Schlafapnoe, Zungenbeissen, Wangenbeissen und Zähneknirschen während der Nacht angewandt. Dazu wird der Unterkiefer während der Nacht in eine protrudierte und geöffnete Parkstellung gebracht und darin gehalten, was aus dem Stand der Technik als Unterkiefer-Protrusions-Schiene bekannt ist. Die meisten der bekannten Schienen sind jeweils aus einer Ober- und Unterkieferschiene die jeweils separat ausgebildet sind und mithilfe eines Scharniers oder nicht festen Verbindungselementes miteinander verbunden sind. Die Anfertigung ist entsprechend aufwendig und der Tragekomfort lässt zu wünschen übrig.

Die US 2014/0076333 offenbart eine Zahnschiene zur Behandlung einer Schlafapnoe, wobei die Schiene aus einem Teil gebildet ist und keine separate Schiene für den Ober- und Unterkiefer vorliegt. Die Position der Zähne des Unter- und Oberkiefers zueinander sind genau definiert, wobei die Zahnschiene überall direkt an den Zähnen anliegt.

Die EP 1 139 905 A1 offenbart einen Mundschutz hauptsächlich für sportliche Anwendungen. Der Mundschutz weist eine stabile äussere U-förmige Schale auf in der eine innere, den Zähnen angepasste Schale angeordnet ist. Eine solche Schiene eignet sich nicht zum Tragen zur Behandlung der erwähnten Schlafprobleme, vielmehr dient eine solche Schiene dem reinen Zahnschutz hauptsächlich bei sportlichen Betätigungen.

Bei den zuvor erwähnten Zahnschienen ist es nachteilig, dass Speichelpfützen unter den Schienen in den Zahnfissuren der Kauflächen entstehen, wie auch in den Interdentalräumen und nicht abfliessen können. Dieses kann zur Folge haben, dass die Bakterien an diesen Stellen übermassig stark ablagern. Beschleunigte Zahnkaries sowie Zahnfleischerkrankungen können dadurch die Folge sein.

Es ist Aufgabe der Erfindung eine Zahnschiene zur Behandlung des Schnarchens, Schlafapnoe, Zungenbeissen, Wangenbeissen und Zähneknirschen während der Nacht vorzuschlagen, welche einen hohen Tragekomfort während des Schlafens gewährleistet und das Bilden von Zahnkaries sowie Zahnfleischerkrankungen oder Ablagerungen vermieden werden kann, indem sich keine Speichelpfützen mehr ansammeln können.

Diese Aufgabe wird erfindungsgemäss dadurch gelöst, dass die Zahnschiene derart ausgebildet ist, dass im Bereich der Kauflächen Speichelkanäle zwischen der Zahnschiene und der Zahnkaufläche vorliegen, die eine permanente Speichelunterspülung der Zahnschiene während des Tragens der Zahnschiene beim Schlafen gewährleisten.

Die erfindungsgemässe Zahnschiene dient dem Arretieren des Unterkiefers, vorzugsweise in einem vordefinierten Protrusions-Verhältnis von Ober- und Unterkiefer. Durch die protrudierte Stellung wird mehr Raum im Rachen geschaffen und gleichzeitig die Halsmuskulatur während des Schlafs über die Zahnreihen des Ober- und Unterkiefers trainiert, was die Behandlung von Schnarchen, Schlafapnoe, Zungenbeissen, Wangenbeissen und Zähneknirschen fördert.

Die erfindungsgemässe Zahnschiene beinhaltet eine Oberkieferschale, eine Unterkieferschale und einen Verbindungssteg, wobei die Ober- und Unterkieferschale über den Verbindungssteg miteinander verbunden sind. Die Ober- und Unterkieferschale sind über den Verbindungssteg fest bzw. unbeweglich zueinander verbunden. Die Zahnschiene weist vorzugsweise ein flexibles Material auf, wodurch die komplette Zahnschiene flexibel ausgebildet ist. Die erfindungsgemässe Zahnschiene ist derart ausgebildet, dass im Bereich der Kauflächen Speichelkanäle zwischen der Zahnschiene und der Zahnkaufläche vorliegen, die eine permanente Speichelunterspülung der Zahnschiene während des Tragens der Zahnschiene beim Schlafen gewährleisten.

Das heisst, dass die Zahnschiene im Bereich der Zahnkauflächen nicht an den Zahnkauflächen anliegt, sondern dort Hohlräume zwischen dem Zahn bzw. der Kaufläche und der Zahnschiene gebildet sind die als Speichelkanäle ausgebildet sind.

Vorzugsweise werden die Speichelkanäle dadurch gebildet, dass die Zahnschiene im Bereich der Fissuren der okklusalen Kauflächen keinen Kontakt mit den Zähnen hat, wodurch unterspülbare Speichelkanäle zwischen den Zahnkauflächen und der Zahnschiene entstehen. Der Speichel kann dadurch jederzeit ungehindert die Zähne umspülen und eine Bakterienbildung unter der eingesetzten Schlafschiene kann verhindert werden.

Vorzugsweise sind die Speichelkanäle zwischen einem Speichelkanalrand an der Zahnschiene und den okklusalen Kauflächen der Zähne gebildet. Der an der Zahnschiene angeordnete Speichelkanalrand ist zur Kaufläche hin gerichtet, liegt jedoch an dieser nicht an, wodurch die Speichelkanäle unter der Zahnschiene gebildet werden. Die Speichelkanäle liegen vorzugswiese im molaren, prämolaren sowie im palatinalen Bereich des Ober- und Unterkiefers.

Als vorteilhaft hat sich gezeigt, wenn die Speichelkanäle entlang der Fissuren der Kauflächen verlaufen. Durch die Fissuren und dem versetzt dazu verlaufenden Speichelkanalrand wird der Speichelkanal unter der Zahnschiene gebildet, der die Umspülung der Zähne und Speichelfluss gewährleistet.

Als bevorzugte Ausgestaltung hat sich gezeigt, wenn die Oberkieferschale, die Unterkieferschale und der Verbindungssteg zusammen als ein Teil ausgebildet sind. Dadurch werden die Kiefergelenke, während dem Schlaf entlastet und bleiben in dieser Stellung geschient, so dass sie sich nicht aus der protrudierten Vorschublage verschieben können.

Vorzugsweise weist die Zahnschiene interdentale Öffnungen auf, wobei die interdentalen Öffnungen zwischen den Zähnen positioniert sind. Die interdentalen Öffnungen verleihen der Zahnschiene eine höhere Flexibilität. Die interdentalen Öffnungen sind vorzugsweise im bukkalen, labialen, palatinalen und/oder lingualen Bereich angeordnet.

Als vorteilhaft hat sich gezeigt, wenn die interdentalen Öffnungen und die im Bereich der Kauflächen angeordneten Speichelkanäle über interdentalen Durchgängen miteinander verbunden sind. Dies ermöglicht die Umspülung der Zähne und gewährleistet, dass der Speichel abfliessen kann.

Als vorteilhaft hat sich gezeigt, wenn die Zahnschiene bukkal angeordnete Öffnungen aufweist.

Vorzugsweise weist die Zahnschiene labial angeordnete Öffnungen auf.

Die bukkalen und labialen Öffnungen sind vorzugsweise auf die Zahnoberfläche ausgerichtet. Vorzugsweise befinden sie sich oberhalb der breitesten Stelle des jeweiligen Zahns in der Unterkieferschale und unterhalb der breitesten Stelle des jeweiligen Zahns in der Oberkieferschale. Durch die Anordnung der bukkalen und labialen Öffnungen an den vorgesehen Stellen wird gewährleistet, dass die Zahnschiene an den Zähnen gut fixiert ist, da die Schiene durch die Öffnungen eine erhöhte Flexibilität und Elastizität erhält was ermöglicht, dass sich die Zahnschiene an die Form des Zahnes anschmiegt. Zudem unterstützen die bukkalen und labialen Öffnungen ebenso die Speichelzirkulation an der Zahnoberfläche.

Es ist vorteilhaft, wenn die Zahnschiene derart ausgebildet ist, dass die Zahnhälse freiliegen. Die freie Zirkulation des Speichels ist ebenfalls dadurch gewährleistet, dass alle Zahnhälse zirkulär der Schiene freiliegen und von der Schiene nicht berührt werden. Es ist vorteilhaft, wenn die Zahnhälse im Ober- und Unterkiefer palatinal, bukkal, lingual und labial freiliegen.

Als bevorzugte Ausführungsform hat sich gezeigt, wenn eine Atmungsöffnung im labialen Bereich zwischen Ober- und Unterkieferschale angeordnet ist. Dies ermöglicht eine gute und leichte Atmung während des Schlafs.

Vorzugsweise ist die Zahnschiene als 3D-Druckteil ausgebildet. Dies gewährleistet eine individuelle Anpassung und Abstimmung der Zahnkonturen an eine Zahnschiene.

Es ist vorteilhaft, wenn die Zahnschiene aus einem Nylon Material hergestellt ist, vorzugsweise einem Medizin zertifizierten biokompatiblen Nylon Material. Dieses Material gewährleistet eine medizinisch unbedenkliche Anwendung wie auch, dass die Oberflächen glatt ausgestellt sind, was einen hohen Tragekomfort gewährleistet und dass dieses Material kaum zerbrechlich ist. Es ist vorteilhaft, wenn die Oberfläche der Zahnschiene chemisch dampfgeglättet ist, was die Schiene versiegelt und ein Eindringen der Bakterien in das Material verhindert.

Alle Ausgestaltungmöglichkeiten sind untereinander frei kombinierbar.

Ein Ausführungsbeispiel der Erfindung wird anhand der Figuren beschrieben, wobei sich die Erfindung nicht nur auf das Ausführungsbeispiel beschränkt. Es zeigen:
Fig. 1 eine dreidimensionale Ansicht einer erfindungsgemässen Zahnschiene an einem Gebiss angeordnet,
Fig. 2 eine dreidimensionale Ansicht einer erfindungsgemässen Zahnschiene,
Fig. 3 den Verlauf der Speichelkanäle im Unterkiefer und
Fig. 4 einen Querschnitt der Zahnschiene an einer Stelle des Kiefers.

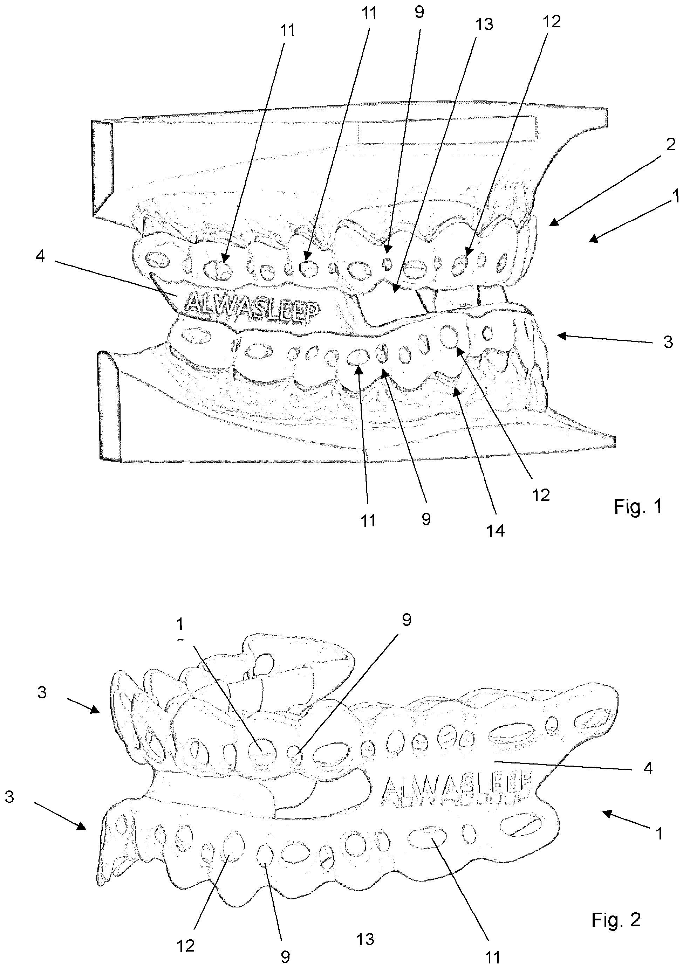

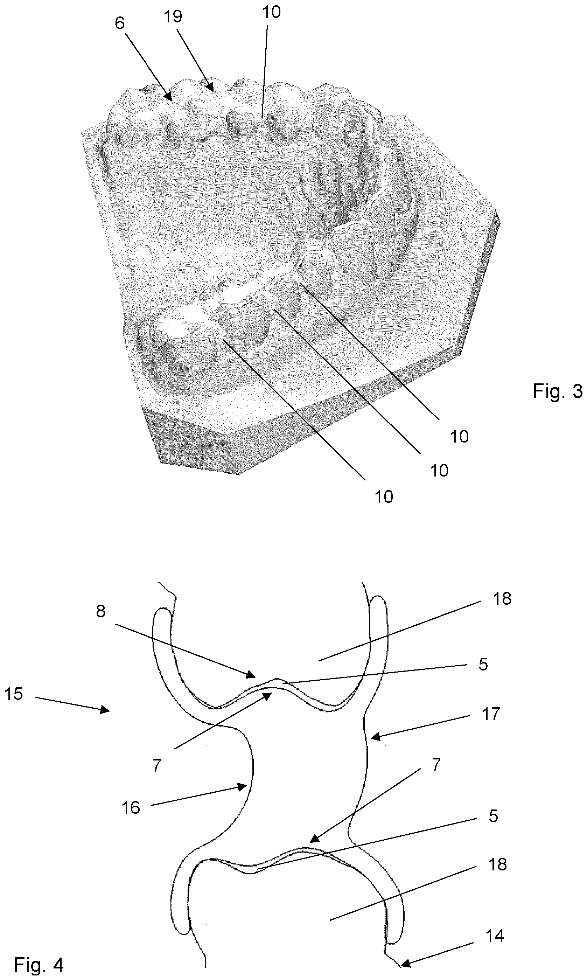

Die in Fig. 1 dargestellte Zeichnung zeigt eine dreidimensionale Ansicht einer erfindungsgemässen Zahnschiene 1 bereits an einem Gebiss angeordnet. Die erfindungsgemässe Zahnschiene 1 weist eine Oberkieferschale 2 und eine Unterkieferschale 3 auf, wobei die beiden Schalen 2, 3 über einen Verbindungssteg 4 fest miteinander verbunden sind. Es ist vorteilhaft, wenn der Verbindungssteg 4 beidseitig ausschliesslich im Bereich der Prämolaren und Molaren angeordnet ist. Die Zahnschiene 1 ist vorzugsweise als einteiliges Teil ausgebildet, das vorzugsweise als 3D-Druckteil hergestellt ist, dies wird in Fig. 2 gut gezeigt. Durch das verwendete Material, vorzugsweise ein Nylon ist die Zahnschiene 1 elastisch verformbar und passt sich flexibel an die Zähne an, ist aber dennoch stabil. Die erfindungsgemässe Zahnschiene 1 bildet, wenn die Zahnschiene 1 am Gebiss angeordnet ist, Speichelkanäle 5 zwischen der Zahnschiene 1 und der Kaufläche 6 der Zähne 18, was gut aus Fig. 4 ersichtlich ist. Durch den Speichelkanal 5 der zwischen der Zahnschiene 1 und dem Zahn 18 gebildet ist, wird eine permanente Speichelunterspülung während des Tragens der Zahnschiene 1 beim Schlafen gewährleistet.

In Fig. 3 ist der verlauf der Speichelkanäle 5 und der interdentalen Durchgänge 10 ersichtlich, entlang denen der Speichel fliessen kann. In der erfindungsgemässen Zahnschiene 1 ist dieser Bereich, der in Fig. 3 als eine Art Füllmasse abgebildet ist, hohl ausgebildet. Fig. 3 zeigt auf, dass die in der Zahnschiene 1 vorliegenden hohl ausgebildeten Speichelkanäle 5 und die hohl ausgebildeten interdentalen Durchgänge 10 zur Herstellung der Zahnschiene 1 mit einem Platzhaltermaterial 19 ausgegossen werden, wobei diese Darstellung gleich als Darstellung des Verlaufs der Speichelkanäle 5 und der interdentalen Durchgänge 10 genutzt werden kann.

Die Speichelkanäle 5 werden durch einen Speichelkanalrand 7, der an der Zahnschiene 1 angeordnet ist und der Kaufläche 6 gebildet, wie das in Fig. 4 dargestellt ist. Die Speichelkanäle 5 verlaufen vorzugsweise entlang der Zahnfissuren 8 in den Kauflächen 6. Dies ist wiederum gut in Fig. 3 ersichtlich, indem mittels dem Platzhaltermaterial 19 der Verlauf der Speichelkanäle 5 aufgezeigt ist. Auch im labialen Bereich wo keine Kauflächen vorliegen verläuft der Speichelkanal 5 entlang der Schneide- und Eckzähne im lingualen und palatinalen Bereich.

Um einerseits die Flexibilität der Zahnschiene 1 zu erhöhen und anderseits die Speichelunterspülung der Zahnschiene bzw. Umspülung der Zähne zu gewährleisten, weist die Zahnschiene 1 vorzugsweise interdentale Öffnungen 9 auf, die zwischen den einzelnen Zähnen 18 positioniert sind, sowohl ins Mundinnere gerichtet wie auch nach aussen hin. Die interdentalen Öffnungen 9 schliessen an die interdentalen Durchgänge 10 an, welche an die Speichelkanäle 5 anschliessen, was einen optimalen Speichelfluss gewährleistet und ein Ansammeln von Bakterien in stehenden Speichelpfützen kann vermieden werden.

Um die Anpassung der Zahnschiene 1 an den Zähnen und deren Flexibilität zu gewährleisten, ist es vorteilhaft, wenn die Zahnschiene 1 bukkale und labiale Öffnungen 11, 12 aufweist, die vorzugsweise auf die Zahnflächen ausgerichtet sind. Zudem ist es vorteilhaft, wenn die bukkalen und labialen Öffnungen 11, 12 ober- oder unterhalb der breitesten Stelle eines jeweiligen Zahnes angeordnet sind. Das heisst, die bukkalen und labialen Öffnungen 11, 12 in der Unterkieferschale 3 liegen oberhalb der breitesten Stelle des jeweiligen Zahnes 18 und in der Oberkieferschale 2 unterhalb der breitesten Stelle des Zahnes 18, dies ermöglicht ein Anschmiegen an die Zähne 18 und gewährleistet dennoch einen guten Halt der Zahnschiene 1 am Gebiss aufgrund der leichten erzeugten Vorspannung durch die Stellen, an denen keine Öffnungen vorgesehen sind. Meist sind die bukkalen und labialen Öffnungen 11, 12 grösser als die interdentalen Öffnungen 9 ausgebildet.

Die Zahnhälse 14 liegen bei allen Zähnen 18 frei, die Schiene 1 endet oberhalb der Zahnhälse 14, was einen hohen Tragekomfort gewährleistet wie auch den Speichelfluss unterstützt. Damit beim Tragen der Zahnschiene 1 eine einwandfreie Atmung gewährleistet werden kann, weist die Zahnschiene im labialen Bereich eine Atmungsöffnung 13 auf.

### Bezugszeichenliste

- 1: Zahnschiene
- 2: Oberkieferschale
- 3: Unterkieferschale
- 4: Verbindungssteg
- 5: Speichelkanal
- 6: Kaufläche
- 7: Speichelkanalrand
- 8: Fissuren
- 9: Interdentale Öffnung
- 10: Interdentaler Durchgang
- 11: Bukkale Öffnung
- 12: Labiale Öffnung
- 13: Atmungsöffnung
- 14: Zahnhals
- 15: Schienenquerschnitt
- 16: Zahnschiene Innenseite Richtung Zunge
- 17: Zahnschiene Aussenseite Richtung Backe
- 18: Zahn
- 19: Platzhaltermaterial

## Patentansprüche

1. Zahnschiene (1) zum Arretieren des Unterkiefers, vorzugsweise in einem vordefinierten Protrusions-Verhätnis von Ober- und Unterkiefer beinhaltend, eine Oberkieferschale (2), eine Unterkieferschale (3) und einen Verbindungssteg (4), wobei die Ober- und Unterkieferschale (2, 3) über den Verbindungssteg (4) miteinander verbunden sind, **dadurch gekennzeichnet, dass** die Zahnschiene (1) derart ausgebildet ist, dass im Bereich der Kauflächen (6) Speichelkanäle (5) zwischen der Zahnschiene (1) und der Zahnkaufläche (6) vorliegen, die eine permanente Speichelunterspülung der Zahnschiene während des Tragens der Zahnschiene (1) beim Schlafen gewährleisten.

2. Zahnschiene (1) nach Anspruch 1, **dadurch gekennzeichnet, dass** die Speichelkanäle (5) zwischen einem Speichelkanalrand (7) an der Zahnschiene (1) und den Kauflächen (6) der Zähne gebildet sind.

3. Zahnschiene (1) nach einem der Ansprüche 1 oder 2, **dadurch gekennzeichnet, dass** die mit der Zahnschiene gebildeten Speichelkanäle (5) entlang der Fissuren (8) in den Zahnkauflächen (6) verlaufen.

4. Zahnschiene (1) nach einem der Ansprüche 1 bis 3, **dadurch gekennzeichnet, dass** die Oberkieferschale (2), die Unterkieferschale (3) und der Verbindungssteg (4) zusammen als ein Teil ausgebildet sind.

5. Zahnschiene (1) nach einem der Ansprüche 1 bis 4, **dadurch gekennzeichnet, dass** die Zahnschiene (1) interdentale Öffnungen (9) aufweist, wobei die interdentalen Öffnungen (9) zwischen den Zähnen positioniert sind.

6. Zahnschiene (1) nach Anspruch 5, **dadurch gekennzeichnet, dass** die interdentalen Öffnungen (9) und die im Bereich der Kauflächen (6) angeordneten Speichelkanäle (5) über interdentale Durchgänge (10) in der Zahnschiene (1) miteinander verbunden sind.

7. Zahnschiene (1) nach einem der Ansprüche 1 bis 6, **dadurch gekennzeichnet, dass** die Zahnschiene (1) bukkal angeordnete Öffnungen (11) aufweist.

8. Zahnschiene (1) nach einem der Ansprüche 1 bis 7, **dadurch gekennzeichnet, dass** die Zahnschiene (1) labial angeordnete Öffnungen (12) aufweist.

9. Zahnschiene (1) nach einem der Ansprüche 1 bis 8, **dadurch gekennzeichnet, dass** die Zahnschiende (1) derart ausgebildet ist, dass die Zahnhälse (14) freiliegen.

10. Zahnschiene (1) nach einem der Ansprüche 1 bis 9, **dadurch gekennzeichnet, dass** eine Atmungsöffnung (13) im labialen Bereich zwischen Ober- und Unterkieferschale (2, 3) angeordnet ist.

11. Zahnschiene (1) nach einem der Ansprüche 1 bis 10, **dadurch gekennzeichnet, dass** die Zahnschiene (1) als 3D-Druckteil ausgebildet ist.

12. Zahnschiene (1) nach einem der Ansprüche 1 bis 11, **dadurch gekennzeichnet, dass** die Zahnschiene (1) aus einem Nylon Material hergestellt ist, vorzugsweise einem Medizin zertifizierten biokompatiblen Nylon Material.
